(19) Europäisches Patentamt
European Patent Office
Office européen des brevets

(11) **EP 1 576 341 B1**

(12) **EUROPÄISCHE PATENTSCHRIFT**

(45) Veröffentlichungstag und Bekanntmachung des Hinweises auf die Patenterteilung:
**20.02.2013 Patentblatt 2013/08**

(21) Anmeldenummer: **03767745.7**

(22) Anmeldetag: **04.12.2003**

(51) Int Cl.:
*G01F 1/704* (2006.01)   *A61B 5/0275* (2006.01)
*A61B 5/028* (2006.01)   *A61M 1/36* (2006.01)
*A61M 1/16* (2006.01)

(86) Internationale Anmeldenummer:
**PCT/EP2003/013730**

(87) Internationale Veröffentlichungsnummer:
**WO 2004/057279 (08.07.2004 Gazette 2004/28)**

(54) **VORRICHTUNG ZUR BESTIMMUNG DES BLUTFLUSSES IN EINEM GEFÄSS EINES PATIENTEN**

DEVICE FOR DETERMINING THE BLOOD FLOW IN A VESSEL OF A PATIENT

DISPOSITIF DE DETERMINATION DU FLUX SANGUIN DANS UN VAISSEAU SANGUIN D'UN PATIENT

(84) Benannte Vertragsstaaten:
**AT BE BG CH CY CZ DE DK EE ES FI FR GB GR HU IE IT LI LU MC NL PT RO SE SI SK TR**

(30) Priorität: **19.12.2002 DE 10259437**

(43) Veröffentlichungstag der Anmeldung:
**21.09.2005 Patentblatt 2005/38**

(73) Patentinhaber: **Fresenius Medical Care Deutschland GmbH**
**61352 Bad Homburg (DE)**

(72) Erfinder: **KRÄMER, Matthias**
**61381 Friedrichsdorf (DE)**

(74) Vertreter: **Ziermann, Oliver**
**Fresenius Medical Care**
**AG & Co. KGaA**
**Global Patents & IP**
**Frankfurter Strasse 6 - 8**
**66606 St. Wendel (DE)**

(56) Entgegenhaltungen:
EP-A- 0 928 614   WO-A-00/24440
WO-A-01/28419   DE-C- 19 528 907
US-A- 3 446 073   US-B1- 6 189 388

**Beschreibung**

Die Erfindung betrifft das Gebiet von Meßverfahren zur Bestimmung des Blutflusses in einer blutführenden Leitung.

Bei Patienten mit Nierenversagen stellt die Hämodialysebehandlung eine Möglichkeit dar, die fehlenden Aufgaben der Niere zu ersetzen. Bei der Hämodialyse wird dem Patienten Blut über eine arterielle Blutleitung entnommen, in einem Blutbehandlungselement gereinigt und über eine venöse Blutleitung wieder zurückgegeben. Das Blutbehandlungselement kann als Hämodialysator ausgeführt sein, bei dem das Blut eine erste Kammer von zwei durch eine semipermeable Membran voneinander getrennten Kammern durchläuft, während die zweite Kammer von Dialysierflüssigkeit durchflossen wird. Durch Beeinflussung der Druckverhältnisse im Dialysator kann dem Blut gezielt Flüssigkeit entzogen werden.

Es ist auch möglich, daß das Blutbehandlungselement als Hämofilter ausgebildet ist. In diesem Fall wird dem Blut über die Membran nur Flüssigkeit entzogen, ohne daß die zweite Kammer von einer Flüssigkeit durchgängig durchströmt wird. Der überwiegende Teil der entzogenen Flüssigkeitsmenge wird dem Patienten über eine Zuführung von Substitutionsflüssigkeit wieder zurückgegeben.

Derartige Behandlungsverfahren benötigen einen ausreichenden Blutfluß von ca. 200 bis 450 ml/min, um innerhalb der mehrere Stunden dauernden Behandlung, die ca. dreimal pro Woche durchgeführt wird, eine ausreichende Reinigung des Blutes zu bewirken. Aus diesem Grunde wird hämodialysepflichtigen Patienten im allgemeinen eine arteriell-venöse Fistel bzw. ein Shunt zwischen einer Arterie und einer Vene gelegt. In diesem Gefäß bildet sich ein ausreichender Blutstrom aus, während das Gefäß gleichzeitig gegenüber den anderen Blutgefässen vergrößerte Ausmaße annimmt, was vorteilhaft für die Punktierung ist.

Der Blutfluß in so einem Gefäß kann mit der Zeit variieren. Insbesondere kann es durch Verengungen zu einer langsamen Verstopfung des Gefässes kommen. Falls der Blutfluß unterhalb der benötigten Blutflußrate im extrakorporalen Kreislauf fällt, wird die Blutreinigungsleistung der Behandlung beeinträchtigt. Meist ist die Verengung des Gefässes in diesem Fall bereits zu weit fortgeschritten, so daß sie nur mit entsprechendem, z.B. chirurgischem Aufwand behoben werden kann. Es ist daher wünschenswert, von einer solchen sich anbahnenden Komplikation früher zu erfahren, wodurch für deren Behebung auch andere Techniken zur Verfügung stehen.

Es sind eine Reihe von Techniken vorgeschlagen worden, den Blutfluß in diesem Gefäß zu messen. Verfahren wie z.B. Ultraschall-Doppler-Systeme, die unabhängig oder mit einem extrakorporalen Blutkreislauf eingesetzt werden (z.B. Weitzel et al., Am. J. Kidney Dis. 38, 935 (2001)), erfordern dazu zusätzliche Geräte und sind kompliziert in der Handhabung. Des weiteren müssen die Messungen durch speziell geschultes Personal durchgeführt werden.

Bei einem anderen bekannten Verfahren wird mit Hilfe einer Infusionspumpe ein Indikator mit konstanter Infusionsrate in ein Gefäß infundiert, wobei flußabwärts Proben aus dem Gefäß genommen werden (Kaye et al., Clinical Nephrology 8, 533 (1977)). Aus der Auswertung der Verdünnungswerte wird auf den Fistelfluß geschlossen. Diese Methode erfordert eine zusätzliche Infusions- sowie Probennahmeeinrichtung sowie den gezielten Einsatz eines Indikators.

Andere Systeme nutzen den extrakorporalen Blutkreislauf des Hämodialysegerätes zur Fistelflußmessung. Die US 5,866,015 beschreibt ein Verfahren, bei die die Blutförderrate im extrakorporalen Kreislauf variiert und der Verlauf der Bluttemperaturen im extrakorporalen Kreislauf gemessen und ausgewertet wird. Auch bei der DE 199 17 197 C1 werden verschiedene Blutflußraten eingestellt, um dann den Verlauf der gemessenen Drücke im extrakorporalen Kreislauf auszuwerten. Nachteilig bei diesen Verfahren ist, daß das Ansteuern verschiedener Blutflußraten aufwendig ist und die Blutbehandlung beeinträchtigt. Des weiteren ist es bei dem in der DE 199 17 197 C1 vorgestellten Verfahren notwendig, den Fistelfluß für einen Teil der Messungen zu unterbrechen.

Nach der Lehre der EP 0 781 161 B1 ist es neben einer Vertauschung der Zugänge an der Fistel notwendig, eine physikalische Eigenschaft des Blutes an der Auslaßseite des extrakorporalen Blutkreislaufes zu ändern, um ein unterscheidbares Blutmerkmal zu erzeugen. Das Ausmaß der Änderung wird dann im Sinne einer Verdünnungskurve ausgewertet. Auch diese Methode erfordert weitere Eingriffe wie z.B. die Injektion einer Indikatorlösung oder eine andere gezielte Veränderung des Blutes. Die Auswertung ist dabei an die Erfassung des gesamten zeitlichen Verlaufes der Änderung gebunden, die über die bolusförmige Änderung integriert werden muß.

Auch anderen Verfahren wie in der WO 02/053212 A1 oder WO 98/17193 A1 beschrieben ist gemeinsam, daß eine gezielte Veränderung des extrakorporalen Blutes sowie der Erfassung deren zeitlichen Verlaufes notwendig ist.

Die US 5,830,365 beschreibt neben der Bestimmung der kardiopulmonaren Rezirkulation auch eine Möglichkeit, mit Hilfe von Rezirkulationsmessungen den Fistelfluß zu bestimmen. Dabei werden jedoch zur Rezirkulationsmessung ebenfalls gezielt bolusartige Veränderungen von Bluteigenschaften bewirkt und ausgewertet.

Die US 4,894,164 beschreibt ein Verfahren und eine Vorrichtung, mit der die Wärmebilanz eines Patienten während einer extrakorporalen Blutbehandlung erfasst und beeinflusst werden können. Eine Bestimmung des Fistelflusses ist dabei nicht vorgesehen.

Der Erfindung liegt daher die Aufgabe zugrunde, eine Vorrichtung zur Bestimmung des Blutflusses in einer blutführenden Leitung bereitzustellen, die auch ohne zusätzliche gezielte bzw. gesteuerte Einwirkungen auf die Bluteigenschaften angewendet werden kann.

Nach der Lehre der Erfindung wird diese Aufgabe durch eine Vorrichtung mit den Merkmalen der Ansprüche 1 und 2 gelöst. Vorteilhafte Ausgestaltungen der Erfindung sind Gegenstand der Unteransprüche.

Die Erfindung findet Anwendung, um den Blutfluß in einem Gefäß eines Patienten zu bestimmen. Hierbei bietet sich die Anwendung vor allem immer dann an, wenn entsprechende Blutleitungen im Rahmen einer extrakorporalen Blutbehandlung sowieso vorliegen. Die Auswertung kann dann unter Ausnutzung bereits vorhandener Komponenten im wesentlichen durch Spezifizierung der Software umgesetzt werden.

Das beschrieben Verfahren kann gleichermaßen allgemein bei Blutleitungen, auch wenn sie außerhalb des menschlichen Körpers verlaufen - z.B. bei in-vitro Anwendungen - zur Anwendung kommen.

Weitere Einzelheiten und Vorteile der Erfindung werden anhand eines in den Zeichnungen dargestellten Ausführungsbeispiels der erfindungsgemäßen Vorrichtung näher beschrieben. Dabei zeigen:

Figur 1 eine schematische Darstellung einer Ausführungsform der erfindungsgemäßen Vorrichtung sowie

Figur 2 verschiedene Fallkonstellationen für den Blutfluß in der blutführenden Leitung, dessen Fluß $Q_F$ bestimmt werden soll.

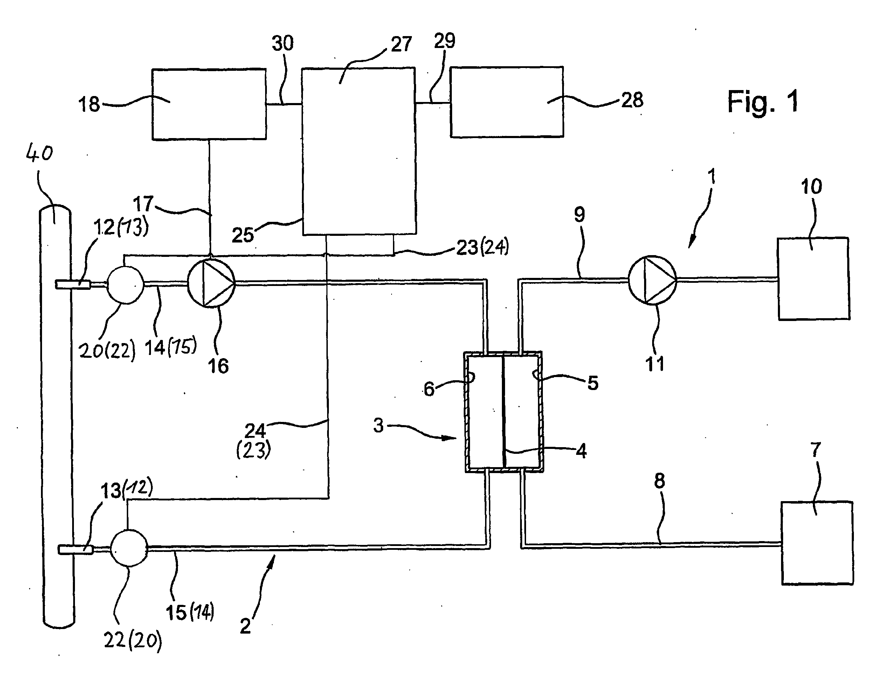

Die in Figur 1 gezeigte Ausführungsform der erfindungsgemäßen Vorrichtung umfaßt einen Dialysekreislauf 1 sowie einen Blutkreislauf 2. Im Dialysekreislauf 1 wird Dialysierflüssigkeit mittels einer Fördereinrichtung 11 von einer Dialysierflüssigkeitsquelle 7 über eine Dialysierflüssigkeitszuführleitung 8, die Dialysierflüssigkeitskammer 5 eines Dia-lysators 3 sowie über Dialysierflüssigkeitsabführleitung 9 zu einem Abfluß 10 gefördert.

In dem Blutkreislauf 2 wird einer blutführenden Leitung 40 Blut an einer ersten Stelle über einen arteriellen Zugang 12 entnommen. An diesen Zugang schließt sich eine arterielle Leitung 14 an, an der ein arterieller Temperatursensor 20 anliegt und in die eine Blutpumpe 16 geschaltet ist. Die arterielle Leitung 14 führt in die Blutkammer 6 des Dialysators 3. Aus dieser Kammer wird das Blut über eine venöse Leitung 15 über einen venösen Zugang 13 der blutführenden Leitung 40 an einer zweiten Stelle zurückgegeben. An der venösen Leitung 15 liegt ein venöser Temperatursensor 22 an.

Die Blutkammer 3 und die Dialysierflüssigkeitskammer 5 sind durch eine semipermeable Membran 4 voneinander getrennt.

Die Vorrichtung weist weiterhin eine Auswerteeinheit 27 auf, die über Meßleitungen 23 und 24 mit dem arteriellen Sensor 20 und dem venösen Sensor 22 zur Erfassung der jeweiligen Temperaturen verbunden ist. Die Auswerteeinheit 27 ist des weiteren über eine Leitung 30 mit einer Steuereinheit 18 zur Ansteuerung der Blutpumpe 16 über eine Leitung 17 verbunden. Über die Steuereinheit 18 kann der Blutpumpe 16 eine Fördergeschwindigkeit vorgegeben werden, die gleichzeitig an die Auswerteeinheit 27 über die Leitung 30 übertragen wird. Die Auswerteeinheit 27 ist über eine Leitung 29 mit einer Anzeigeeinrichtung 28 verbunden, auf der die Meß- und Steuerdaten sowie Auswertungsergebnisse dargestellt werden können.

Die Erfindung basiert auf der Beobachtung, daß es zur Bestimmung des Leitungsflußes $Q_F$ in der Leitung 40 keiner gezielten Manipulation der Bluteigenschaften z.B. in der venösen Leitung 15 bedarf. Es ist vielmehr ausreichend, die von der Leitung 40 über die arterielle Leitung 14 abgeführte sowie über die venöse Leitung 15 zugeführte Nettorate $dX/dt$ einer Größe X zu bestimmen, die von einer physikalisch-chemischen Größe Y des Blutes abgeleitet ist. Die Nettorate $dX/dt$, die aus der Differenz der arteriellen Rate $dX_A/dt$ und der venösen Rate $dX_V/dt$ bestimmt wird, läßt sich für den Fall ausreichender zeitlicher Konstanz während eines Meßintervalls mit Hilfe der Werte $Y_A$ und $Y_V$ der physikalisch-chemischen Eigenschaft Y in der arteriellen und venösen Leitung berechnen. Die Nettorate $dX/dt$ kann dann zur Ableitung des Leitungsflußes $Q_F$ herangezogen werden.

Für die Nettorate $dX/dt$ gilt in dieser Situation

$$\frac{dX}{dt} = \frac{dX_V}{dt} - \frac{dX_A}{dt} = Q_B(Y_V - Y_A) \qquad (1),$$

wobei $Q_B$ der Blutfluß in der arteriellen bzw. venösen Leitung ist.

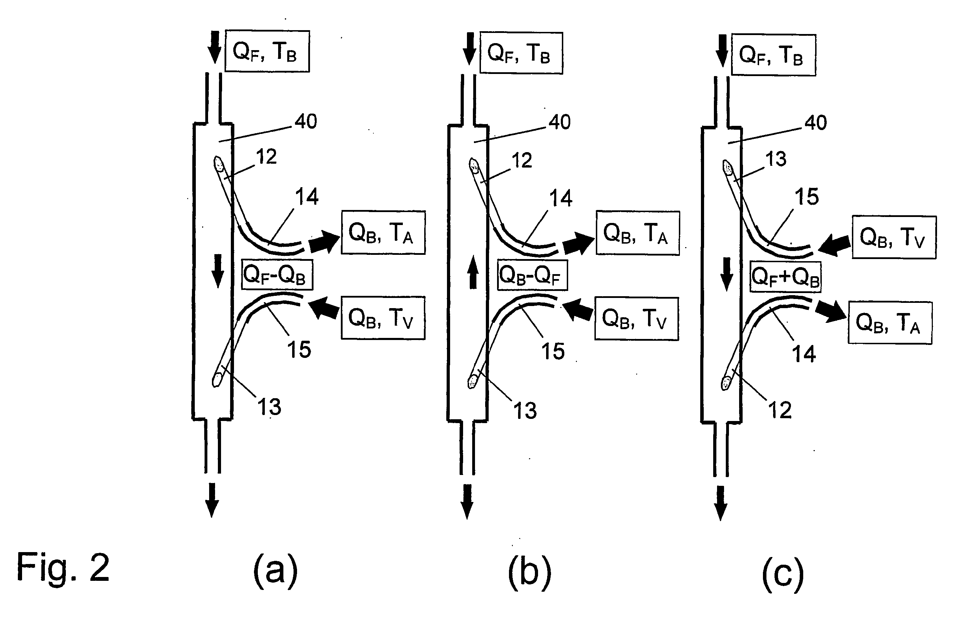

Für den zu bestimmenden Blutfluß $Q_F$ und den Blutfluß $Q_B$ im Blutkreislauf 2 sind verschiedene Fälle möglich, die in Figur 2 näher dargestellt sind. In den Figuren 2a und 2b liegt die Abzweigung 12 der arteriellen Leitung 14 stromaufwärts der Abzweigung 13 der venösen Leitung 15. Figur 2a betrifft den Fall, daß der Blutfluß $Q_B$ kleiner als der zu messende Fluß $Q_F$ ist. In diesem Fall verbleibt ein Fluß $Q_F-Q_B$ zwischen der arteriellen und venösen Abzweigung, und es kommt zu keiner unmittelbaren Rezirkulation in der Leitung 40 von über die venöse Leitung 15 zurückgeführten Blut zur arteriellen Leitung 14. In diesem Fall ist die vorliegende Erfindung zunächst nicht anwendbar.

Wird jedoch ein Fluß $Q_B$ vorgegeben, der den Fluß $Q_F$ übersteigt (Figur 2b), so kommt es zur Rezirkulation in der blutführenden Leitung 40. Diese Situation kann in solchen Fällen kontrolliert herbeigeführt werden, wenn der zu messende Fluß $Q_F$ nur in einem gewissen Wertebereich erwartet wird. Dieser kann dann durch $Q_B$ überschritten werden, wodurch eine Rezirkulation erzwungen wird. In diesem Fall setzt sich der Blutfluß $Q_B$ aus zwei Komponenten zusammen:

$$Q_B = R \cdot Q_B + Q_F \qquad (2).$$

Die erste Komponente betrifft den rezirkulierenden Teil und der zweite Teil den der Leitung 40 zufließenden Teil. Der Rezirkulationsfaktor R gibt dabei den prozentualen Anteil des Rezirkulationsflußes am Blutfluß $Q_B$ an.

Der rezirkulierende Anteil liefert jedoch keinen Anteil an der Nettorate dX/dt, da er der blutführenden Leitung 40 gleichermaßen zu- und abgeführt wird. Zu dieser Nettorate dX/dt kann nur der zweite Teil einen Beitrag leisten:

$$\frac{dX}{dt} = Q_F (Y_V - Y_B) \qquad (3),$$

wobei $Y_B$ die physikalisch-chemische Eigenschaft in der Leitung 40 vor der ersten Abzweigung 12 ist. Nach Auflösung nach $Q_F$ ergibt sich aus Gleichung (3)

$$Q_F = \frac{\dfrac{dX}{dt}}{Y_V - Y_B} = \frac{Q_B(Y_V - Y_A)}{Y_V - Y_B} \qquad (4).$$

Falls Y die Wärmeenergie pro Volumen Blut und X die Wärmeenergie E des Blutes in der blutführenden Leitung 40 ist, wird aus Gleichung (4)

$$Q_F = \frac{\dfrac{dE}{dt}}{c_E \rho_B (T_V - T_B)} = \frac{Q_B(T_V - T_A)}{(T_V - T_B)} \qquad (5a),$$

wobei $T_A$, $T_V$ und $T_B$ die Temperaturen des Blutes in der arteriellen Leitung 20, in der venösen Leitung 22 und in dem der blutführenden Leitung 40 zufließenden Blut sind. Die Wärmekapazität des Blutes ist mit $c_E$ und die Dichte des Blutes mit $\rho_B$ angegeben, wobei angenommen wurde, daß sie in allen Leitungen gleich ist.

Würde Y die Konzentration c eines Stoffes und X die Stoffmenge C dieses Stoffes in der blutführenden Leitung 40 sein, so lautet die Gleichung (5a) entsprechende Gleichung (5b) mit entsprechenden Indizes:

$$Q_F = \frac{\dfrac{dC}{dt}}{(c_V - c_B)} = \frac{Q_B(c_V - c_A)}{(c_V - c_B)} \qquad (5b).$$

Das Verfahren kann durch die Ausführungsform in Figur 1, in der eine Hämodialysevorrichtung gezeigt ist, folgendermaßen durchgeführt werden: Es hat sich gezeigt, daß bei einer Dialysebehandlung während eines Meßzeitintervalles von einigen Sekunden bis wenigen Minuten davon ausgegangen werden kann, daß die Temperaturen des Blutes in den Leitungen 14, 15 und 40 ausreichend konstant bleiben. Die Auswerteeinrichtung 27 speichert die während des Meßzeitintervalles mit den Sensoren 20 und 22 ermittelten Temperaturen $T_A$ in der arteriellen Leitung 14 und $T_V$ in der venösen Leitung 15. Zur Steigerung der Genauigkeit werden dabei die Sensoren 20 und 22 so dicht wie möglich an die Abzweigungen 12 bzw. 13 angeordnet. Aufgrund der Wegstrecke, die das Blut vom arteriellen Sensor 20 bis zum venösen Sensor 22 - insbesondere über den Dialysator 3 - zurücklegt, hat sich überraschend gezeigt, daß die Temparturen $T_A$ und $T_V$ im Anwendungsfall praktisch immer inhärent ausreichend verschieden sind, um eine Messung zu ermöglichen.

Von der Steuereinheit 18 wird der Blutflußwert $Q_B$ an die Auswerteeinheit gemeldet. Dieser ist von der Steuereinheit 18 ausreichend hoch gewählt worden, damit der Fall gemäß Figur 2b eintritt.

Damit die Auswerteeinheit 27 nun mit Hilfe der Gleichung (5a) den Fluß $Q_F$ in der blutführenden Leitung 40 bestimmen kann, wird bei dieser Ausführungsform folgende zusätzliche Maßnahme ergriffen: Unmittelbar vor oder nach der Messung der Temperaturen $T_A$ und $T_V$ stellt die Steuereinheit den Blutfluß auf einen Wert $Q_{B2} < Q_B$, für den der Fall zutrifft, der in Figur 2a gezeigt ist. Bei der Dialyse wäre z.B. $Q_{B2}$=150 ml/min geeignet. Dann erfaßt die Auswerteeinheit 27 den Temperaturwert $T_A$ für den Blutfluß $Q_{B2}$. Dieser Wert, der ebenfalls von der Auswerteeinheit 27 abgespeichert wird, entspricht dem Temperaturwert $T_B$ in Gleichung (5a). Damit sind alle Größen in Gleichung (5a) bekannt, und die Auswerteeinheit 27 kann den Fluß $Q_F$ bestimmen. Ggf. wird er dann auf der Anzeigeeinrichtung 28 zur Anzeige gebracht.

Eine andere Ausführungsvariante macht von der Situation Gebrauch, wie sie in Figur 2c dargestellt ist. In diesem Fall zweigt die arterielle Leitung 14 stromabwärts der venösen Leitung 15 von der blutführenden Leitung 40 ab. Diese Konstellation ist in Figur 1 in Klammern dargestellt. Bei der Vorrichtung nach Figur 1 kann dies leicht dadurch bewerkstelligt werden, daß die Förderrichtung der Blutpumpe 16 umgekehrt wird. Es wäre genauso möglich, die Anschlüsse 12 und 13 zu vertauschen. In diesem Zusammenhang wird explizit auf die Offenbarung der DE 195 28 907 C1 verwiesen, bei der eine Weichenschaltung für diesen Fall vorgestellt wird. Diese kann manuell, aber auch automatisch von der Steuereinheit 18 angesteuert werden.

In Analogie zu Gleichung (2) gilt für den Fall gemäß Figur 2c:

$$Q_B = R \cdot Q_B + (1-R)Q_B \qquad (6).$$

In diesem Fall gilt weiter

$$R = \frac{Q_B}{Q_B + Q_F} \qquad (7).$$

Wieder liefert nur der nichtrezirkulierende, zweite Anteil in Gleichung (6) einen Beitrag zur Nettorate dX/dt, die für den Fall nach Figur 2c mit $dX_{rec}/dt$ bezeichnet werden soll.

Die Gleichung (3) lautet dementsprechend

$$\frac{dX_{rec}}{dt} = \frac{dX_{V,rec}}{dt} - \frac{dX_{A,rec}}{dt} = (1 - \frac{Q_B}{Q_B + Q_F})Q_B(Y_{V,rec} - Y_B) \qquad (8).$$

Nach $Q_F$ aufgelöst wird aus Gleichung (8)

$$Q_F = \frac{Q_B \dfrac{dX_{rec}}{dt}}{Q_B(Y_{V,rec} - Y_B) - \dfrac{dX_{rec}}{dt}} = \frac{Q_B(Y_{V,rec} - Y_{A,rec})}{Y_{A,rec} - Y_B} \qquad (9).$$

Nun kann in analoger Weise wie für den Fall nach Figur 2b verfahren werden. Die Meßgröße $Y_B$ kann dabei ebenfalls analog bestimmt werden, wobei die Anschlüsse gemäß der Figur 2a vorgenommen werden müssen. Dies kann manuell oder über die Steuereinheit 18 gesteuert durchgeführt werden, indem die Förderrichtung der Blutpumpe 16 umgedreht wird oder eine entsprechende Weichenschaltung verwendet wird. Die zu den Gleichungen (5a) und (5b) analogen Zusammenhänge ergeben sich durch Einsetzen der Größen Temperatur T (wobei noch mit der spezifischen Wärmekapzität $c_E$ und der Dichte $\rho_B$ zu multiplizieren ist) bzw. Konzentration c in Gleichung (9).

In einer besonders bevorzugten, dritten Ausführungsvariante wird zunächst eine erste Messung in der Konstellation nach Figur 2a oder 2b für die Nettorate dX/dt durchgeführt. Dann wird durch Umkehrung der Förderrichtung der Blutpumpe 16 oder durch eine entsprechende Weichenschaltung eine zweite Messung der Nettorate $dX_{rec}/dt$ in der Konstellation nach Figur 2c durchgeführt. Wird nun Gleichung (8) durch Gleichung (1) geteilt, so ergibt sich Gleichung (10)

$$\frac{\dfrac{dX_{rec}}{dt}}{\dfrac{dX}{dt}} = \frac{Q_F}{Q_B + Q_F} \frac{Y_{V,rec} - Y_B}{Y_V - Y_A} \qquad (10).$$

Wird der Blutfluß $Q_B$ so klein gewählt, daß der Fall gemäß Figur 2a für die Messung von dX/dt vorliegt, so ist $Y_A = Y_B$. In Gleichung (10) eingesetzt wird daraus für diesen Fall:

$$Q_F = \frac{Z}{1 - Z} Q_B \qquad (11a),$$

wobei

$$Z = \frac{\dfrac{dX_{rec}}{dt}}{\dfrac{dX}{dt}} \frac{Y_V - Y_A}{Y_{V,rec} - Y_A} = \frac{Y_{V,rec} - Y_{A,rec}}{Y_{V,rec} - Y_A} \qquad (11b).$$

In dieser Ausführungsvariante berechnet die Auswerteeinheit 27 den Fluß $Q_F$ nach den Gleichungen (11a) und (11b), wozu die Auswerteeinheit 27 die einzelnen Meßwerte wie bei den vorangegangenen Ausführungsvarianten zunächst abspeichert.

Die zu den Gleichungen (5a) und (5b) analogen Zusammenhänge ergeben sich nunmehr wiederum durch Einsetzen der Größen Temperatur T (wobei noch mit der spezifischen Wärmekapzität $c_E$ und der Dichte $\rho_B$ zu multiplizieren ist) bzw. Konzentration c in Gleichung (11 b). Sollten die Messungen für dX/dt und $dX_{rec}/dt$ bei unterschiedlichen Blutflüssen $Q_B$ und $Q_{B,rec}$ durchgeführt werden, so kann Gleichung (11) entsprechend angepaßt werden.

Das Gleichungssystem (11 a) und (11 b) kann nun unter bestimmten Bedingungen weiter vereinfacht werden. Bei der in Figur 1 gezeigten Vorrichtung durchfließt das Blut die Blutkammer 6 des Dialysators 3. Dabei kommt es zum Stoff- und Energieaustausch mit der Dialysierflüssigkeit in der Dialysierflüssigkeitskammer 5. Die beiden Flüssigkeiten durchfließen - wie in der Hämodialyse allgemein üblich - den Dialysator in gegenläufiger Richtung, wobei der Dialysierflüssigkeitfluß im allgemeinen größer als der Blutfluß gewählt wird. Dabei tritt oft - je nach den vorliegenden Flußverhältnissen und den verwendeten Dialysatoren - insbesondere für die Temperatur die Situation ein, daß das Blut am Ausgang der Blutkammer 6 die Temperatur der Dialysierflüssigkeit am Eingang der Dialysierflüssigkeitskammer 5 annimmt.

Wird nun die Temperatur in der Dialysierflüssigkeitszuführleitung 8 während der Meßphase, die Sekunden bis höchstens wenige Minuten andauert, konstant ge halten, so bleibt auch die Temperatur des Blutes in der venösen Leitung 15 konstant. Dabei haben kleinere Abweichungen der Temperatur des Blutes in der arteriellen Leitung 14 keinen Einfluß. Dies bedeutet, daß die Temperaturen $T_V$ und $T_{V,rec}$ in Gleichung (11 b) identisch sind. (Hierbei muß für die in Figur 1 gezeichnete Ausführungsform der Dialysatfluß für die Bestimmung von $dX_{rec}/dt$ umgekehrt werden. Bei Verwendung einer Weichenschaltung, bei der die Flußbedingungen im Dialysator 3 beibehalten werden, ist dies nicht notwendig.)

Damit kürzen sich der Zähler und der Nenner des zweiten Bruchs nach dem ersten Gleichheitszeichen identisch. In Gleichung (11a) eingesetzt wird daraus Gleichung (12):

$$Q_F = Q_B \frac{\dfrac{dX_{rec}}{dt}}{\dfrac{dX}{dt} - \dfrac{dX_{rec}}{dt}} \tag{12}.$$

Die nach Gleichung (1) bestimmten Nettoraten $dX/dt$ bzw. $dX_{rec}/dt$ können in diesem Fall besonders einfach verwendet werden, um den Blutfluß in der blutführenden Leitung 40 zu bestimmen. Dies gilt immer dann, wenn das Kriterium $Y_V = Y_{V,rec}$ erfüllt ist.

Die hier genannten Ausführungen sind unter der Annahme abgeleitet worden, daß der Blutfluß in der arteriellen Leitung 14 und der venösen Leitung 15 identisch ist. Bei der Hämodialyse kann es in bestimmten Fällen zu kleinen Abweichungen von dieser Annahme kommen, wenn den arteriellen bzw. venösen Leitungen Flüssigkeit durch Ultrafiltration entnommen wird. Es liegt jedoch im handwerklichen Können des Fachmanns, die Gleichungen dieser Situation anzupassen. Neben der Blutflußrate in einer der Leitung muß dann lediglich zusätzlich der Ultrafiltrationsfluß erfaßt werden.

Ähnliches gilt im Falle der Messung an Gefäßen eines Patienten für die sogenannte kardiopulmonäre Rezirkulation. Bei der kardiopulmonären Rezirkulation gelangt von der venösen Leitung 15 an die Leitung 40 abgegebenes Blut mit den Eigenschaften $Y_V$ zur arteriellen Leitung 14, in dem es über den Blutkreislauf des Patienten direkt rezirkuliert, ohne einen ausreichenden Stoffwechsel- oder Temperaturausgleich in anderen Körperbereichen zu erfahren. Dieser Anteil ist jedoch im allgemeinen relativ klein.

Mit der Erfindung steht eine Vorrichtung zur Verfügung, bei der mit minimalen Aufwand der Blutfluß in einer Leitung bestimmt werden kann, von dem eine arterielle und eine venöse Leitung abzweigen. Die Messungen können innerhalb kurzer Zeit durchgeführt werden, wodurch eine Beeinflußung einer eventuell gleichzeitigen Blutbehandlung auf einem vernachlässigbaren Niveau gehalten werden kann. Eine gezielte Zugabe von Indikatoren ist nicht erforderlich.

## Patentansprüche

1. Vorrichtung zur Messung des Blutflusses in einem Gefäß eines Patienten, insbesondere einer arteriell-venösen Fistel bzw. eines Shuntes, mit
   einem extrakorporalen Blutkreislauf mit einem Dialysator zur Durchführung einer extrakorporalen Blutbehandlung, der eine von dem Gefäß (40) abzweigende arterielle Leitung (14), mit der Blut von dem Gefäß abgeführt wird und die in eine Blutkammer des Dialysators führt, und eine in das Gefäß (40) mündende venöse Leitung (15), mit der das Blut aus der Blutkammer dem Gefäß zugeführt wird, umfasst,
   arteriellen (20) und venösen Meßmitteln (22) zum Bestimmen einer für ein Meßintervall zeitlich konstanten physikalisch-chemischen Größe Y des Blutes in der arteriellen Leitung (14) mit Wert $Y_A$ und in der venösen Leitung (15) mit Wert $Y_V$,
   einer mit den arteriellen (20) und venösen Meßmitteln (22) verbundenen Auswerteeinheit (27), die geeignet ist, die Nettorate $dX/dt$ einer von der physikalisch-chemischen Größe Y abgeleiteten Größe X in bzw. aus dem Gefäß (40) während des Meßintervalls als Differenz der über die arterielle Leitung (14) abgeführten Rate $dX_A/dt$ und der über die venöse Leitung (15) zugeführten Rate $dX_V/dt$ aus den Werten $Y_A$ und $Y_V$ ohne zusätzliche gesteuerte Einwirkungen auf die Bluteigenschaften zu bestimmen und die Nettorate $dX/dt$ zur Bestimmung des Blutflusses $Q_F$ zu verwenden,
   wobei Mittel (18) vorgesehen sind, den Blutfluß $Q_B$ in der arteriellen (14) und der venösen Leitung (15) zu erfassen oder vorzugeben,
   wobei die physikalisch-chemische Größe Y die Wärmeenergie pro Volumen Blut und die davon abgeleitete Größe X die Wärmeenergie E des Blutes in dem Gefäß (40) ist,
   und wobei zur Bestimmung der Nettowärmeenergierate $dE/dt$ die Meßmittel (20, 22) in der arteriellen Leitung ($T_A$) und der venösen Leitung ($T_V$) Temperatursensoren sind und die Auswerteeinheit (27) geeignet ist, die Nettowärmeenergierate aufgrund der Beziehung

EP 1 576 341 B1

$$\frac{dE}{dt} = \frac{dE_V}{dt} - \frac{dE_A}{dt} = c_E \rho_B Q_B (T_V - T_A)$$

zu bestimmen, wobei $c_E$ die spezifische Wärmekapazität und $\rho_B$ die Dichte des Blutes ist.

2. Vorrichtung zur Messung des Blutflusses in einem Gefäß eines Patienten, insbesondere einer arteriell-venösen Fistel bzw. eines Shuntes, mit

einem extrakorporalen Blutkreislauf mit einem Dialysator zur Durchführung einer extrakorporalen Blutbehandlung, der eine von dem Gefäß (40) abzweigende arterielle Leitung (14), mit der Blut von dem Gefäß abgeführt wird und die in eine Blutkammer des Dialysators führt, und eine in das Gefäß (40) mündende venöse Leitung (15), mit der das Blut aus der Blutkammer dem Gefäß zugeführt wird, umfasst,

arteriellen (20) und venösen Meßmitteln (22) zum Bestimmen einer für ein Meßintervall zeitlich konstanten physikalisch-chemischen Größe Y des Blutes in der arteriellen Leitung (14) mit Wert $Y_A$ und in der venösen Leitung (15) mit Wert $Y_V$,

einer mit den arteriellen (20) und venösen Meßmitteln (22) verbundenen Auswerteeinheit (27), die geeignet ist, die Nettorate $dX/dt$ einer von der physikalisch-chemischen Größe Y abgeleiteten Größe X in bzw. aus dem Gefäß (40) während des Meßintervalls als Differenz der über die arterielle Leitung (14) abgeführten Rate $dX_A/dt$ und der über die venöse Leitung (15) zugeführten Rate $dX_V/dt$ aus den Werten $Y_A$ und $Y_V$ ohne zusätzliche gesteuerte Einwirkungen auf die Bluteigenschaften zu bestimmen und die Nettorate $dX/dt$ zur Bestimmung des Blutflusses $Q_F$ zu verwenden,

wobei Mittel (18) vorgesehen sind, den Blutfluß $Q_B$ in der arteriellen (14) und der venösen Leitung (15) zu erfassen oder vorzugeben,

wobei die physikalisch-chemische Größe Y die Konzentration c eines Stoffes im Blut und X die Stoffmenge C dieses Stoffes in dem Gefäß (40) ist,

und wobei zur Bestimmung der Nettostoffmengenrate $dC/dt$ die Meßmittel (20, 22) in der arteriellen Leitung $c_A$ und der venösen Leitung $c_V$ Konzentrationssensoren sind und die Auswerteeinheit (27) geeignet ist, die Nettostoffmengenrate aufgrund der Beziehung

$$\frac{dC}{dt} = \frac{dC_V}{dt} - \frac{dC_A}{dt} = Q_B (c_V - c_A)$$

zu bestimmen.

3. Vorrichtung nach Anspruch 1 oder 2, **dadurch gekennzeichnet dass** die Mittel zur Erfassung des Blutflusses QB aus einem Flußsensor bestehen, der mit der Auswerteeinheit (27) verbunden ist.

4. Vorrichtung nach Anspruch 3, **dadurch gekennzeichnet, daß** die Mittel zur Erfassung des Blutflusses $Q_B$ aus einer Steuereinheit (18) bestehen, die zum Vorgeben einer Fördergeschwindigkeit einer Blutpumpe (16), die in der arteriellen (14) und/oder der venösen Leitung (15) angeordnet ist, und die mit der Auswerteeinheit (27) verbunden ist.

5. Vorrichtung nach einem der vorhergehenden Ansprüche, **dadurch gekennzeichnet, daß** die arterielle Leitung (14) stromaufwärts der venösen Leitung (15) von dem Gefäß (40) abzweigt und daß die Auswerteeinheit (27) geeignet ist, die Bestimmung des Blutflusses $Q_F$ aufgrund der Beziehung

$$Q_F = \frac{\dfrac{dX}{dt}}{Y_V - Y_B}$$

vorzunehmen, wobei $Y_B$ die physikalisch-chemische Größe in dem Gefäß (40) stromaufwärts der Abzweigung (12)

8

**EP 1 576 341 B1**

der arteriellen Leitung (14) ist.

6. Vorrichtung nach einem der vorhergehenden Ansprüche, **dadurch gekennzeichnet, daß** die arterielle Leitung (14) stromabwärts der venösen Leitung (15) von dem Gefäß (40) abzweigt, wobei die Nettorate mit $dX_{rec}/dt$ und die physikalisch-chemische Größe in der venösen Leitung mit $Y_{v,rec}$ bezeichnet werden, und daß die Auswerteeinheit (27) geeignet ist, die Bestimmung des Blutflusses $Q_F$ aufgrund der Beziehung

$$Q_F = \frac{Q_B \dfrac{dX_{rec}}{dt}}{Q_B (Y_{V,rec} - Y_B) - \dfrac{dX_{rec}}{dt}}$$

vorzunehmen, wobei $Y_B$ die physikalisch-chemische Größe in dem Gefäß (40) stromaufwärts der Abzweigung (13) der venösen Leitung (15) ist.

7. Vorrichtung nach einem der vorhergehenden Ansprüche, **dadurch gekennzeichnet, daß** die Auswerteeinheit (27) geeignet ist, sowohl die Nettorate $dX/dt$ bei stromaufwärtsseitiger Abzweigung der arteriellen Leitung (14) gegenüber der venösen Leitung (15) von dem Gefäß (40) als auch die Nettorate $dX_{rec}/dt$ bei stromabwärtsseitiger Abzweigung der arteriellen Leitung (12) gegenüber der venösen Leitung (15) von dem Gefäß (40) bei gleichem Blutfluß $Q_B$ und daraus den Blutfluß $Q_F$ nach der folgenden Beziehung zu bestimmen:

$$Q_F = \frac{Z}{1-Z} Q_B \quad \text{mit} \quad Z = \frac{\dfrac{dX_{rec}}{dt}}{\dfrac{dX}{dt}} \frac{Y_V - Y_A}{Y_{V,rec} - Y_A} .$$

8. Vorrichtung nach einem der vorhergehenden Ansprüche, **dadurch gekennzeichnet, daß** der extrakorporale Blutkreislauf Teil eines Hämodialysegerätes ist.

9. Vorrichtung nach einem der vorhergehenden Ansprüche, **dadurch gekennzeichnet, daß** die Vorrichtung eine Anzeigeeinrichtung (28) aufweist, die geeignet ist, den Blutfluß $Q_F$ anzuzeigen.

**Claims**

1. A device for measuring the blood flow in a patient's vessel, in particular an arteriovenous fistula and/or a shunt, having an extracorporeal blood circulation with a dialyzer for performing an extracorporeal blood treatment, said blood circulation comprising an arterial line (14) that branches off from the vessel (40), and carries the blood away from the vessel and leads into a blood chamber of the dialyzer, and comprising a venous line (15) which opens into the vessel (40) and returns the blood from the blood chamber back to the vessel,
arterial measuring means (20) and venous measuring means (22) for determining a physicochemical variable Y of the blood having the value $Y_A$ in the arterial line (14) and having the value $Y_V$ in the venous line (15), said variable being constant over time for a measurement interval,
an analysis unit (27), which is connected to the arterial measuring means (20) and to the venous measuring means (22) and is suitable for determining the net rate $dX/dt$ of a variable X, derived from the physicochemical variable Y, into and/or out of the vessel (40) during the measurement interval as the difference between the rate $dX_A/dt$ withdrawn through the arterial line (14) and the rate $dX_V/dt$ supplied through the venous line (15), from the values $Y_A$ and $Y_V$, without any additional controlled influences on the blood properties and using the net rate $dX/dt$ to determine the blood flow $Q_F$,
wherein means (18) are provided, which detect or determine the blood flow $Q_B$ in the arterial line (14) and in the venous line (15),
wherein the physicochemical variable Y is the thermal energy per unit of volume of blood, and the variable X derived from the former is the thermal energy E of the blood in the vessel (40),
and wherein to determine the net thermal energy rate dE/dt, the measuring means (20, 22) in the arterial line ($T_A$) and the venous line ($T_V$) comprise temperature sensors, and the analysis unit (27) is suitable for determining the net thermal energy rate, on the basis of the equation

$$\frac{dE}{dt} = \frac{dE_V}{dt} - \frac{dE_A}{dt} = c_E \rho_B Q_B (T_V - T_A)$$

where $C_E$ is the specific thermal capacity and $\rho_B$ is the density of the blood.

2. A device for measuring the blood flow in a patient's vessel, in particular an arteriovenous fistula and/or a shunt, having an extracorporeal blood circulation with a dialyzer for performing an extracorporeal blood treatment, said blood circulation comprising an arterial line (14) that branches off from the vessel (40) and carries the blood away from the vessel and leads into a blood chamber of the dialyzer, and comprising a venous line (15) which opens into the vessel (40) and returns the blood from the blood chamber back to the vessel,
arterial measuring means (20) and venous measuring means (22) for determining a physicochemical variable Y of the blood having the value $Y_A$ in the arterial line (14) and having the value $Y_V$ in the venous line (15), said variable being constant over time for a measurement interval,
an analysis unit (27), which is connected to the arterial measuring means (20) and to the venous measuring means (22) and is suitable for determining the net rate dX/dt of a variable X, derived from the physicochemical variable Y, into and/or out of the vessel (40) during the measurement interval as the difference between the rate $dX_A$/dt withdrawn through the arterial line (14) and the rate $dX_V$/dt supplied through the venous line (15), from the values $Y_A$ and $Y_V$ without any additional controlled influences on the blood properties and using the net rate dX/dt to determine the blood flow $Q_F$,
wherein means (18) are provided, which detect or determine the blood flow $Q_B$ in the arterial line (14) and in the venous line (15),
wherein the physicochemical variable Y is the concentration c of a substance in the blood, and X is the quantity C of this substance in the vessel (40),
and wherein to determine the net substance quantity rate dC/dt, the measuring means (20, 22) in the arterial line $c_A$ and in the venous line $c_V$ are concentration sensors, and the analysis unit (27) is suitable for determining the net substance quantity rate on the basis of the equation

$$\frac{dC}{dt} = \frac{dC_V}{dt} - \frac{dC_A}{dt} = Q_B (c_V - c_A) \ .$$

3. The device according to Claim 1 or 2, **characterized in that** the means for detecting the blood flow QB consist of a flow sensor connected to the analysis unit (27).

4. The device according to Claim 3, **characterized in that** the means for detecting the blood flow $Q_B$ consist of a control unit (18), which is provided for determining a delivery rate of a blood pump (16), which is arranged in the arterial line (14) and/or in the venous line (15) and is connected to the analysis unit (27).

5. The device according to any one of the preceding claims, **characterized in that** the arterial line (14) branches off from the vessel (40) upstream from the venous line (15), and the analysis unit (27) is suitable for performing the determination of the blood flow $Q_F$ on the basis of the equation:

$$Q_F = \frac{\dfrac{dX}{dt}}{Y_V - Y_B}$$

where $Y_B$ is the physicochemical variable in the vessel (40) upstream from the branch (12) in the arterial line (14).

6. The device according to any one of the preceding claims, **characterized in that** the arterial line (14) branches off from the vessel (40) downstream from the venous line (15), wherein the net rate $dX_{rec}/dt$ and the physicochemical variable in the venous line are referred to as $Y_{V,rec}$ and the analysis unit (27) is suitable for performing the determination of the blood flow $Q_F$ on the basis of the equation:

$$Q_F = \frac{Q_B \dfrac{dX_{rec}}{dt}}{Q_B(Y_{V,rec} - Y_B) - \dfrac{dX_{rec}}{dt}}$$

wherein $Y_B$ is the physicochemical variable in the vessel (40) upstream from the branch (13) in the venous line (15).

7. The device according to any one of the preceding claims, **characterized in that** the analysis unit (27) is suitable for determining both the net rate $dX/dt$ in the case of an upstream branch in the arterial line (14) with respect to the venous line (15) from the vessel (40) as well as the net rate $dX_{rec}/dt$ in the downstream branch of the arterial line (14) with respect to the venous line (15) from the vessel (40) at the same blood flow $Q_B$ and from this to determine the blood flow $Q_F$ according to the following equation:

$$Q_F = \frac{Z}{1-Z} Q_B \quad \text{where} \quad Z = \frac{\dfrac{dX_{rec}}{dt}}{\dfrac{dX}{dt}} \frac{Y_V - Y_A}{Y_{V,rec} - Y_A}$$

8. The device according to any one of the preceding claims, **characterized in that** the extracorporeal blood circulation is part of a hemodialysis machine.

9. The device according to any one of the preceding claims, **characterized in that** the device has a display unit (28) suitable for displaying the blood flow $Q_F$.

**Revendications**

1. Dispositif pour mesurer le débit sanguin dans un vaisseau d'un patient, en particulier dans une fistule artéro-veineuse respectivement un shunt, comprenant
une circulation sanguine extracorporelle avec un dialyseur pour effectuer un traitement du sang extracorporel qui comprend une ligne artérielle (14) dérivée du vaisseau (40) avec laquelle du sang est évacué du vaisseau et laquelle conduit dans une chambre sanguine du dialyseur, et une ligne veineuse (15) débouchant dans le vaisseau (40) avec laquelle le sang sort de la chambre sanguine et est amené au vaisseau,
des moyens de mesure artérielle (20) et veineuse (22) pour déterminer une valeur Y physico-chimique du sang constante temporellement pour un intervalle de mesure, dans la ligne artérielle (14) avec la valeur $Y_A$ et dans la ligne veineuse (15) avec la valeur $Y_V$,
une unité d'évaluation (27) reliée aux moyens de mesure artérielle (20) et veineuse (22) qui est appropriée pour déterminer le débit net $dX/dt$ d'une valeur X dérivée de la valeur Y physico-chimique dans le, respectivement, hors du vaisseau (40) pendant l'intervalle de mesure en tant que différence du débit $dX_A/dt$ évacué via la ligne artérielle (14) et du débit $dX_V/dt$ amené via la ligne veineuse (15) à partir des valeurs $Y_A$ et $Y_V$ sans effets commandés supplémentaires sur les propriétés sanguines et pour employer le débit net $dX/dt$ pour déterminer le débit sanguin $Q_F$, sachant des moyens (18) sont prévus pour saisir ou fournir le débit sanguin $Q_B$ dans la ligne artérielle (14) ou veineuse (15),
sachant que la valeur Y physico-chimique est l'énergie calorifique par volume de sang et la valeur X qui en est dérivée est l'énergie calorifique E du sang dans le vaisseau (40),

et sachant que pour déterminer le taux d'énergie calorifique net dE/dt, les moyens de mesure (20, 22) dans la ligne artérielle ($T_A$) et la ligne veineuse ($T_V$) sont des capteurs de température et l'unité d'évaluation (27) est appropriée pour déterminer le taux d'énergie calorifique net par la relation

$$\frac{dE}{dt} = \frac{dE_V}{dt} - \frac{dE_A}{dt} = c_E \rho_B Q_B (T_V - T_A)$$

sachant que $C_E$ est la capacité calorifique spécifique et $P_B$ la densité sanguine.

2. Dispositif pour mesurer le débit sanguin dans un vaisseau d'un patient, en particulier une fistule artéro-veineuse respectivement un shunt, comprenant

une circulation sanguine extracorporelle avec un dialyseur pour effectuer un traitement du sang extracorporel qui comprend une ligne artérielle (14) dérivée du vaisseau (40) avec laquelle du sang est évacué du vaisseau et laquelle conduit dans une chambre sanguine du dialyseur, et une ligne veineuse (15) débouchant dans le vaisseau (40) avec laquelle le sang sort de la chambre sanguine et est amené au vaisseau,

des moyens de mesure artérielle (20) et veineuse (22) pour déterminer une valeur Y physico-chimique du sang constante temporellement pour un intervalle de mesure, dans la ligne artérielle (14) avec la valeur $Y_A$ et dans la ligne veineuse (15) avec la valeur $Y_V$,

une unité d'évaluation (27) reliée aux moyens de mesure artérielle (20) et veineuse (22) qui est appropriée pour déterminer le débit net dX/dt d'une valeur X dérivée de la valeur Y physico-chimique dans le, respectivement, hors du vaisseau (40) pendant l'intervalle de mesure en tant que différence du débit dXA/dt évacué via la ligne artérielle (14) et du débit $dX_V$/dt amené via la ligne veineuse (15) à partir des valeurs $Y_A$ et $Y_V$ sans effets commandés supplémentaires sur les propriétés sanguines et pour employer le débit net dX/dt pour déterminer le débit sanguin $Q_F$, sachant des moyens (18) sont prévus pour saisir ou fournir le débit sanguin $Q_B$ dans la ligne artérielle (14) ou veineuse (15),

sachant que la valeur Y physico-chimique est la concentration c d'une substance dans le sang et X la quantité C de cette substance dans le vaisseau (40),

et sachant que pour déterminer la quantité de substance nette dC/dt, les moyens de mesure (20, 22) dans la ligne artérielle $C_A$ et la ligne veineuse Cv sont des capteurs de concentration et l'unité d'évaluation (27) est appropriée pour déterminer la quantité de substance nette par la relation

$$\frac{dC}{dt} = \frac{dC_V}{dt} - \frac{dC_A}{dt} = Q_B (c_V - c_A)$$

3. Dispositif selon la revendication 1 ou 2, **caractérisé en ce que** les moyens de détection du débit sanguin $Q_B$ sont composés d'un capteur de débit qui est relié à l'unité d'évaluation (27).

4. Dispositif selon la revendication 1 ou 2, **caractérisé en ce que** les moyens de détection du débit sanguin $Q_B$ sont composés d'une unité de commande (18) qui est disposée pour donner une vitesse de transport d'une pompe sanguine (16) placée dans la ligne artérielle (14) et/ou veineuse (15) et est reliée à l'unité d'évaluation (27).

5. Dispositif selon l'une des revendications précédentes, **caractérisé en ce que** la ligne artérielle (14) est dérivée du vaisseau (40) en amont de la ligne veineuse (15) et que l'unité de mesure (27) est appropriée pour déterminer le débit sanguin $Q_F$ par la relation

$$Q_F = \frac{\dfrac{dX}{dt}}{Y_V - Y_B}$$

sachant que $Y_B$ est la valeur physico-chimique dans le vaisseau (40) en amont de la dérivation (12) de la ligne artérielle (14).

6. Dispositif selon l'une des revendications précédentes, **caractérisé en ce que** la ligne artérielle (14) est dérivée du vaisseau (40) en aval de la ligne veineuse (15), sachant que le débit net est désigné par $DX_{rec}/dt$ et la valeur physico-chimique dans la ligne veineuse est désignée par $Y_{V,rec}$ et que l'unité d'évaluation (27) est appropriée pour déterminer le débit sanguin $Q_F$ par la relation

$$Q_F = \frac{Q_B \dfrac{dX_{rec}}{dt}}{Q_B(Y_{V,rec} - Y_B) - \dfrac{dX_{rec}}{dt}}$$

sachant que $Y_B$ est la valeur physico-chimique dans le vaisseau (40) en amont de la dérivation (13) de la ligne veineuse (15).

7. Dispositif selon l'une des revendications précédentes, **caractérisé en ce que** l'unité d'évaluation (27) est appropriée pour déterminer tant le débit net $dX/dt$ en cas de dérivation en amont de la ligne artérielle (14) par rapport à la ligne veineuse (15) du vaisseau (40), que le débit net $dX_{rec}/dt$ en cas de dérivation en aval de la ligne artérielle (14) par rapport à la ligne veineuse (15) du vaisseau (40) pour un même débit sanguin $Q_B$ et d'en déterminer le débit sanguin $Q_F$ selon la relation suivante :

$$Q_F = \frac{Z}{1-Z} Q_B \quad \text{avec} \quad Z = \frac{\dfrac{dX_{rec}}{dt}}{\dfrac{dX}{dt}} \frac{Y_V - Y_A}{Y_{V,rec} - Y_A}$$

8. Dispositif selon l'une des revendications précédentes, **caractérisé en ce que** la circulation sanguine extracorporelle fait partie d'un appareil d'hémodialyse.

9. Dispositif selon l'une des revendications précédentes, **caractérisé en ce que** le dispositif présente un dispositif d'affichage (28) qui est approprié pour afficher le débit sanguin $Q_F$.

Fig. 1

EP 1 576 341 B1

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5866015 A
- DE 19917197 C1
- EP 0781161 B1
- WO 02053212 A1
- WO 9817193 A1
- US 5830365 A
- US 4894164 A
- DE 19528907 C1